# EUROPEAN PATENT SPECIFICATION

(11) **EP 3 032 944 B1**
(45) Date of publication and mention of the grant of the patent: **18.10.2017**
(21) Application number: 14758700.0
(22) Date of filing: 08.08.2014
(51) Int. Cl.: A01K 67/033

(54) **CAGE FOR BREEDING INSECTS, RACK, SYSTEM AND METHOD**
KÄFIG ZUR ZUCHT VON INSEKTEN SOWIE GESTELL, SYSTEM UND VERFAHREN
CAGE POUR ÉLEVER DES INSECTES, RÂTELIER, SYSTÈME ET PROCÉDÉ

(30) Priority: 13.08.2013 NL 2011300
(43) Date of publication of application: 22.06.2016
(73) Proprietor: Protix Biosystems B.V., 5107 NC Dongen (NL)
(72) Inventor: AARTS, Kees Wilhelmus Petrus, 5126 CT Gilze (NL)
(74) Representative: Patentwerk B.V.
(86) International application number: PCT/NL2014/050554
(87) International publication number: WO 2015/023178

(56) References cited:
- DE-A1- 2 747 040
- FR-A- 1 410 620
- US-A- 5 158 497

## Description

The present invention relates to a cage for breeding insects, a rack for storing a plurality of these cages, and a method for breeding insects.

Insects and their larvae are used as an animal protein feed, and for this purpose they are produced on a large scale. Systems for producing and/or breeding insects on large scales are well known in the art, and in particular influencing the mating, breeding and growth of the insects by controlling the light in a breeding space for the insects is known. For instance, US patent US 5158497 discloses an illuminated dome for controlled mixture of flying insects.

The French patent FR 1 410 620 describes a cage according to the preamble of claim 1.

The German patent DE 2747040 describes a method for breeding insects.

The systems known in the art have however, several disadvantages, which make them inefficient in multiple ways. It is therefore a purpose of the present invention to provide a cage for breeding insects, a rack for storing a plurality of these cages, and a method for breeding insects, that takes away the disadvantages of the prior art.

The invention thereto proposes a cage for breeding insects according to claim 1, comprising: a space, limited by walls, for housing said insects and/or pupae thereof wherein the walls are impenetrable by the insects, at least one of the walls is at least partly light translucent and at least one of the walls comprises an openable part, for inserting insects. A bottom wall of the comprises a gutter for conducting fluid to a fluid exit opening.

The cage according to the invention forms a device for breeding insects, that can be easily handled and stored, and that provides a facility to influence the breeding, mating and laying of eggs, by regulating at least a property of the light that is added through the at least partly light transparent wall.

The cage may for instance have a rectangular shape and be arranged as a drawer for a rack containing multiple cages. Typically it may have a length between 1 and 3 meters, a width between 1 and 3 meters, and a height between 0,2 and 1,5, in particular between 0,2 and 0,3 meters. Bigger cages are found to be less effective due to the controllability of lighting, climate, and collectability of the eggs. The at least partly light translucent wall and/or any of the other walls may be gas-permeable to permit fresh air to enter the cage. The openable part provides the possibility to enter a number of insects or pupae or cocoons, and to close the cage after placement.

In particular, the openable part comprises a tray. Such tray can be moved between a closed position and an opened position, wherein the tray closes the cage at least in the closed position, but permits to add insects in the opened position. It may preferably be arranged such that it is configured to receive insects in the opened position, while closing an opening in the cage, and to transfer the insects (or larvae) to the interior of the cage when the tray is moved to the closed position.

In a further embodiment, the tray is removable. This enables to switch trays quickly, and to swap an empty tray for a filled one, for entering new larvae or insects. The at least one tray may be arranged to close an opening in a bottom of the cage, that is: at least in a closed position of the tray. Such configuration leads to a lower chance of insects exiting the cage when a tray is removed or exchanged. Such openable part may be accessible from a side of the cage, which enables addition of larvae while the cage is stored in a rack.

Preferably, an upper wall comprises the at least partly light translucent part. This enables to lighten the cage from the top-side, and in particular, to incorporate lighting means in a rack for cages. However, other walls may be at least partly translucent as well. The at least partly translucent part can for instance be formed by a gauze or netting, but alternatively, a translucent part is used which has a opal glass or milk glass or a coloured glass for filtering a certain portion from the light that enters the cage. The glass may be selected in correspondence with the lighting source, in order to remove undesired components from the light from the lighting source.

In a further embodiment, at least one of the walls comprises a slot for inserting an egg depository device. Such slot may be arranged at the same side as the openable part for inserting insects, for convenient handling when the cage is stored in a rack. The use of a depository device results in easy harvesting of the eggs.

The invention also relates to a cage provided with an above mentioned egg depository device, which may be a sheet-like element of any material that encourages the insects to lay their eggs on. Such egg depository device may further comprise at least one gas inlet accessible from the outside of the cage when the device is placed in the cage; and at least one gas outlet device, arranged at the inside of the cage when the device is placed in the cage. In a preferred embodiment, the egg depository device is arranged to divide a gas stream offered at the input evenly over said egg depository device, in order to efficiently use its egg-laying area. Thereto, the egg depository device may comprise for instance a gas permeable material, like a sponge, a plate with holes or small cravices or a specially designed structure like a cilinder.

The gas inlet and outlet device may be used for inserting a gas that in particular is a fertilizer gas, or any other organic or synthetic odorous gas, which has proven to stimulate the insects to lay their eggs at the depository device, instead of laying them at a random location in the cage.

In yet another embodiment of the present invention, at least one of the walls comprises an entrance for inserting a fluid. Such fluid is used to clean the cage, and can be applied when the cage is stored in a rack.

In again another aspect, the invention relates to an arrangement of a plurality of cages as described above, wherein the cages are arranged such that they can be illuminated by a lighting source. The cages may for this purpose for instance be stacked, mounted on or against a wall or be arranged in a rack.

Such rack may comprise at least one light source, wherein the lighting source is arranged such that it can illuminate the interior of a cage through the at least one at least partly light translucent wall.

Preferably, the rack comprises multiple light sources, each arranged such that it can illuminate the interior of a cage through the at least one at least partly light translucent wall. The rack may be designed such that multiple cages can be stacked inside, with individual light sources above each of the stacked cages. A distance in height direction of 0,1 to 1 meter may be provided for this purpose in the rack.

The light sources may thereto be arranged in spaces between openings for cages, and for example be chosen from a group of Led, TL or LEP, each having their own specific effect on the mating of the insects. Combinations of various light sources are thinkable too, wherein each of the light sources can be controlled separately. The rack may further comprise or be coupled to a control system for controlling on/off cycles of the light sources. These cycles may comprise for instance switching the lights off while the flies hatch, and after a number of days, for instance about 7 days, switching the lights off for an hour, and then on again for two hours. In yet a further embodiment, the rack also comprises a control system for controlling moisture and/or temperature, which may be coupled to the control system for the light, or form part of the same control system. One or more sprinklers may be arranged in the vicinity, in particular above, or in a cage, for adding moisture to the cage.

The control system may provide a combined control of light, temperature and moisture.

The invention will now be elucidated into more detail with reference to the following figures; herein:
- Figure 1 shows a cage according to the present invention;
- Figure 2 shows a rack according to the present invention with a device for adding a fluid;
- Figure 3 shows the position of light assemblies and cages in a rack according to the present invention;
- Figure 4 shows a hall with racks according to the present invention; and
- Figure 5 shows a schematic overview of a method according to the present invention.

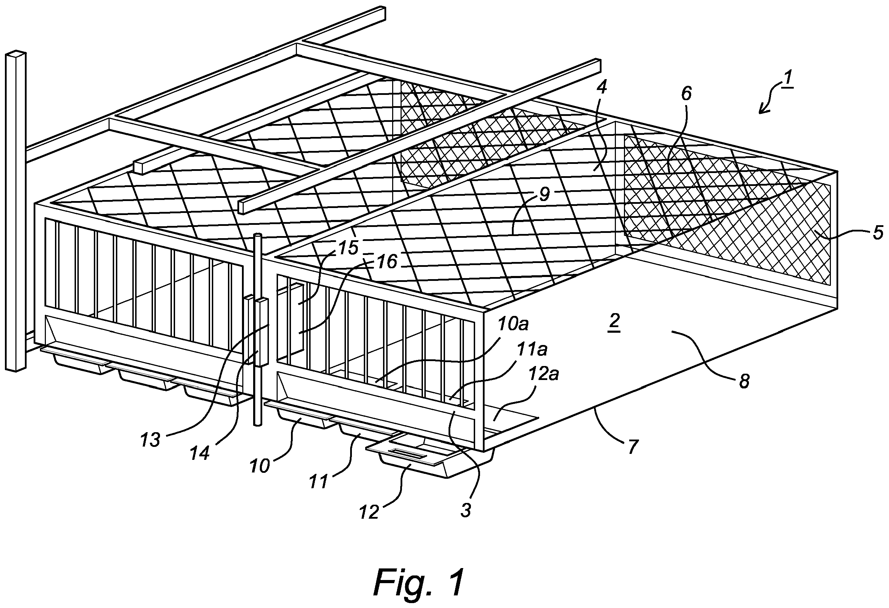

Figure 1 shows two cages 1 according to the present invention, for breeding insects, comprising a space 2, limited by walls 3, 4, 5, 6, 7, 8 (invisible), for housing said insects and/or larvae thereof.

The walls are impenetrable by the insects by an gauze, which is so fine that it is not visible in the drawing and the upper wall 4 is partly light translucent, that is, through the gauze and netting 9, which latter serves for supporting the gauze. A front wall 3 comprises openable parts 10, 11, 12, each formed by a tray, for inserting insects. Instead of a rigid tray, which may be made from a plastics or a metal, a vacuum-cleaner cartridge-like paper tray is also thinkable. The tray can be removed in order to be replaced by a new one. In a closed position, the trays close a respective opening 10a, 11a, 12a in a bottom of the cage, at least in a closed position of the tray. The trays are accessible from the front wall 3 of the cage.

The front wall 3 is further provided with a slot 13 for inserting an egg depository device 14. This egg depository device 14 comprises at least one gas inlet 15 accessible from the outside of the cage 1 when the device 14 is placed in the cage 1; and at least one gas outlet 16, arranged at the inside of the cage 1 when the device 14 is placed in the cage 1.

The egg depository device 14 comprises a gas permeable material, like a sponge.

The cage 1 further has a valve or cover for permitting access to a fluid entrance device, for cleaning the cage.

Also a sprinkler nozzle may form part of the cage, or form part of its environment, like a rack in which the cage is placed, in order to add moisture to the interior.

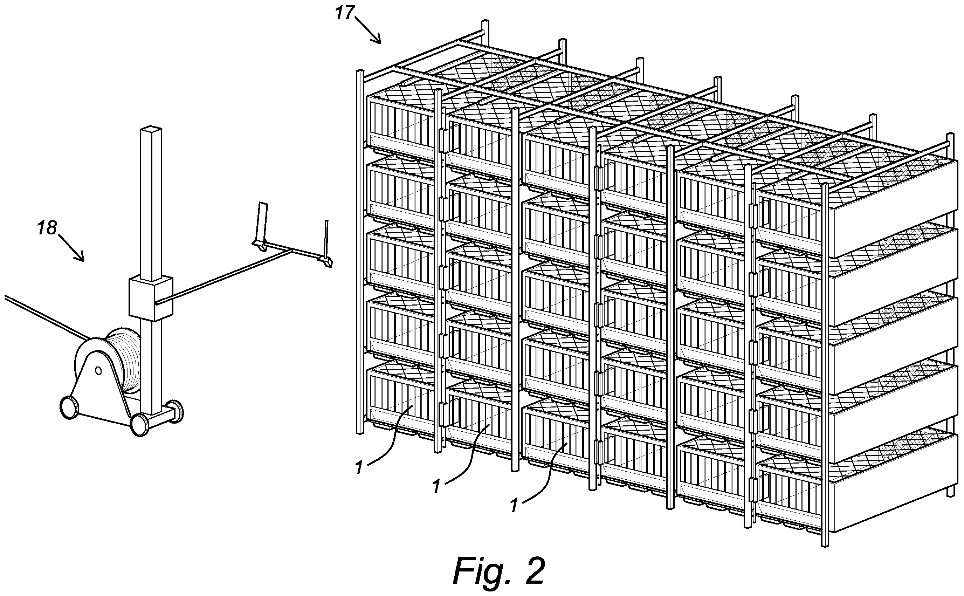

Figure 2 shows a rack 17 according to the present invention, with a device 18 for adding a fluid. Such fluid serves to clean the cages 1, which thereto comprise an entrance (not visible) for inserting a fluid, as well as a gutter (not visible) for conducting fluid to a fluid exit opening.

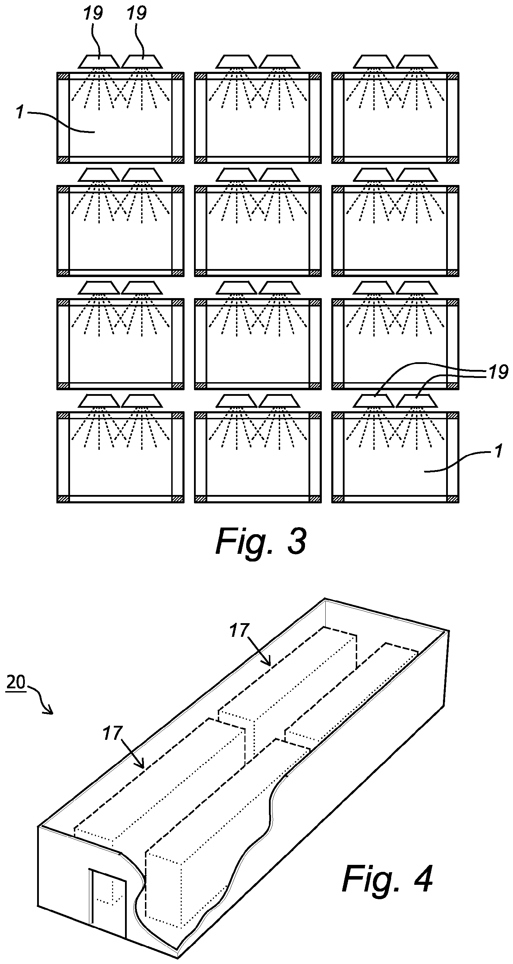

Figure 3 shows the position of light assemblies 19 and cages 1 in a rack according to the present invention. From the figure, it is clear that there are lighting assemblies for each separate cage. Not depicted are sprinklers that may be present above each cage too, for controlling moisture.

Figure 4 shows a hall 20 with racks 17 according to the present invention, which may comprise a moisture conditioning system.

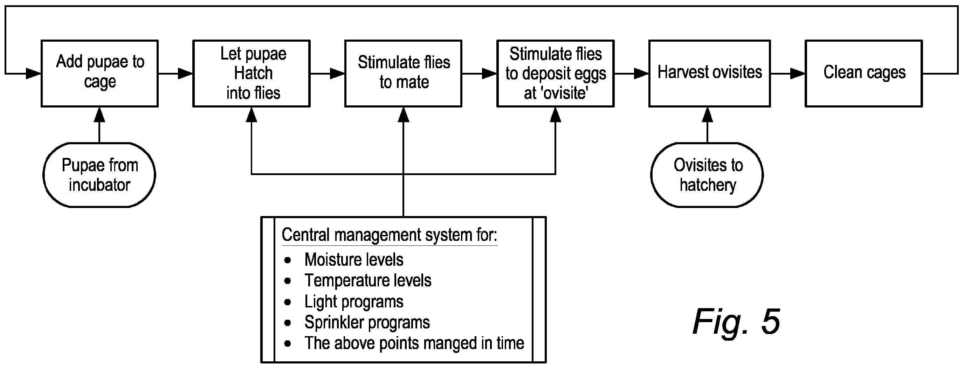

Figure 5 shows a schematic overview of a method according to the present invention, which comprises the following steps.
1. A cage is designed to house a certain amount of flies.
2. A certain amount of pupae is added to the cage after which they hatch inside the cage.
3. A certain amount of hatched flies fills the cage.
4. The flies are stimulated to mate in order to ensure fertility of the eggs.
5. The female flies are stimulated to lay their eggs on an element so that easy harvesting of eggs is possible (egg deposit systems)
6. After a certain amount of harvesting moments the flies are left to die in the cage
7. The cage is cleaned by removing all the dead flies and leftover living flies and the cage is further cleaned for new run of cycle (step 1-7)

The above examples and embodiments are exemplary only and do not limit in any sense the scope of protection of the present application as defined by the following claims.

## Claims

1. Cage (1) for breeding insects, comprising:
- a space (2), limited by walls (3, 4, 5, 6, 7, 8), for housing said insects and/or pupae and/or cocoons thereof;
wherein:
- the walls are impenetrable by the insects;
- at least one (4) of the walls is at least partly light translucent; and
- at least one of the walls comprises an openable part (10, 11, 12), for inserting insects and/or pupae and/or cocoons, **characterised in that** a bottom wall comprises a gutter for conducting fluid to a fluid exit opening.

2. Cage (1) according to claim 1, wherein the openable part comprises at least one tray (10, 11, 12), in particular wherein the at least one tray (10, 11, 12) is removable.

3. Cage (1) according to claim 2, wherein the at least one tray (10, 11, 12) is arranged to close an opening in a bottom of the cage (1), at least in a closed position of the tray (10, 11, 12).

4. Cage (1) according to any of the preceding claims wherein the openable part (10, 11, 12) is accessible from a side of the cage (1).

5. Cage (1) according to any of the preceding claims, wherein an upper wall (4) comprises the at least partly light translucent part, which is in particular formed by a gauze or netting.

6. Cage (1) according to any of the preceding claims, wherein at least one of the walls (3, 4, 5, 6, 7, 8) comprises a slot (13) for inserting an egg depository device (14), comprising at least one gas inlet accessible from the outside of the cage when the device (14) is placed in the cage (1); and at least one gas outlet device, arranged at the inside of the cage when the device is placed in the cage.

7. Cage (1) according to claim 6, wherein the egg depository device (14) comprises a gas permeable material, like a sponge.

8. Cage (1) according to any of the preceding claims, wherein at least one of the walls (3, 4, 5, 6, 7, 8) comprises an entrance for inserting a fluid.

9. Combination of a plurality of cages (1) according to any of the preceding claims, and lighting sources, the cages and lighting sources arranged such that at least the partly light translucent wall of each cage can be illuminated by a lighting source.

10. Rack for holding a plurality of cages (1) according to anyone of claims 1 to 8 comprising at least one lighting source, wherein the lighting source is arranged such that it can illuminate the interior of a cage through the at least one at least partly light translucent wall.

11. Rack according to claim 10, comprising a control system for controlling moisture and/or temperature.

12. Method for breeding insects, comprising:
- Providing a cage (1) according to any of claims 1-8;
- Adding a certain amount of pupae is to the cage (1);
- Having the pupae hatch inside the cage (1) ;
- Stimulating flies to mate in order to ensure fertility of the eggs;
- Stimulating female flies to lay their eggs on a depository device (14) so that easy harvesting of eggs is possible.

13. Method according to claim 12, comprising the steps of:
- After a certain amount of harvesting moments, leaving the flies left to die in the cage;
- Cleaning the cage (1) by removing all the dead flies and leftover living flies.

14. Method according to claim 13, comprising repeating all steps with a new amount of flies.

## Patentansprüche

1. Käfig (1) zur Zucht von Insekten, umfassend:
- einen Raum (2), der durch Wände (3, 4, 5, 6, 7, 8) begrenzt ist, um die Insekten und/oder deren Puppen und/oder Kokons aufzunehmen;
wobei:
- die Wände für die Insekten undurchdringlich sind;
- mindestens eine (4) der Wände mindestens teilweise lichtdurchlässig ist; und
- mindestens eine der Wände einen Teil (10, 11, 12) umfasst, der geöffnet werden kann, um Insekten und/oder Puppen und/oder Kokons hineinzugeben, **dadurch gekennzeichnet, dass** eine Bodenwand eine Rinne umfasst, um Fluid zu einer Fluidaustrittöffnung zu leiten.

2. Käfig (1) nach Anspruch 1, wobei der Teil, welcher geöffnet werden kann, mindestens eine Wanne (10, 11, 12) umfasst, wobei die mindestens eine Wanne (10, 11, 12) entfernbar ist.

3. Käfig (1) nach Anspruch 2, wobei die mindestens eine Wanne (10, 11, 12) so angeordnet ist, dass eine Öffnung in einem Boden des Käfigs (1) mindestens in einer geschlossenen Position der Wanne (10, 11, 12) geschlossen wird.

4. Käfig (1) nach einem der vorhergehenden Ansprüche, wobei der Teil (10, 11, 12), der geöffnet werden kann, von einer Seite des Käfigs (1) aus zugänglich ist.

5. Käfig (1) nach einem der vorhergehenden Ansprüche, wobei eine obere Wand (4) den mindestens teilweise lichtdurchlässigen Teil umfasst, der insbesondere durch eine Gaze oder ein Netzmaterial gebildet ist.

6. Käfig (1) nach einem der vorhergehenden Ansprüche, wobei mindestens eine der Wände (3, 4, 5, 6, 7, 8) einen Schlitz (13) zum Hineingeben einer Eihinterlegungsvorrichtung (14) umfasst, die mindestens einen Gaseinlass, der von der Außenseite des Käfigs zugänglich ist, wenn die Vorrichtung (14) in dem Käfig (1) platziert wird; und mindestens eine Gasauslassvorrichtung umfasst, die an der Innenseite des Käfigs angeordnet ist, wenn die Vorrichtung in dem Käfig platziert wird.

7. Käfig (1) nach Anspruch 6, wobei die Eihinterlegungsvorrichtung (14) gasdurchlässiges Material umfasst, wie einen Schwamm.

8. Käfig (1) nach einem der vorhergehenden Ansprüche, wobei mindestens eine der Wände (3, 4, 5, 6, 7, 8) einen Eingang zum Hineingeben eines Fluids umfasst.

9. Kombination aus einer Vielzahl von Käfigen (1) nach einem der vorhergehenden Ansprüche und Beleuchtungsquellen, wobei die Käfige und Beleuchtungsquellen so angeordnet sind, dass mindestens die teilweise lichtdurchlässige Wand jedes Käfigs durch eine Beleuchtungsquelle beleuchtet werden kann.

10. Gestell zum Halten einer Vielzahl von Käfigen (1) nach einem der Ansprüche 1 bis 8, umfassend mindestens eine Beleuchtungsquelle, wobei die Beleuchtungsquelle so angeordnet ist, dass sie das Innere eines Käfigs durch die mindestens eine teilweise lichtdurchlässige Wand beleuchten kann.

11. Gestell nach Anspruch 10, das ein Steuersystem zur Steuerung von Feuchtigkeit und/oder Temperatur umfasst.

12. Verfahren zur Zucht von Insekten, umfassend:
- Bereitstellen eines Käfigs (1) nach einem der Ansprüche 1 bis 8;
- Hinzufügen einer bestimmten Menge an Puppen zu dem Käfig (1);
- Schlüpfen lassen der Puppen im Inneren des Käfigs (1) ;
- Stimulieren von Fliegen zur Paarung, um Fertilität der Eier zu gewährleisten;
- Stimulieren von weiblichen Fliegen, damit sie ihre Eier auf einer Hinterlegungsvorrichtung (14) ablegen, so dass leichtes Ernten der Eier möglich ist.

13. Verfahren nach Anspruch 12, umfassend die folgenden Schritte:
- Sterben lassen der Fliegen in dem Käfig nach einer bestimmten Menge an Erntevorgängen;
- Reinigen des Käfigs (1) durch Entfernen aller toten Fliegen und übrig gebliebenen lebenden Fliegen.

14. Verfahren nach Anspruch 13, umfassend Wiederholen aller Schritte mit einer neuen Menge an Fliegen.

## Revendications

1. Cage (1) pour l'élevage d'insectes, comprenant :
- un espace (2), limité par des parois (3, 4, 5, 6, 7, 8), pour loger lesdits insectes et/ou des nymphes et/ou des cocons de ceux-ci ;
dans laquelle :
- les parois sont impénétrables par les insectes ;
- au moins l'une (4) des parois est au moins en partie translucide ; et
- au moins l'une des parois comprend une partie ouvrable (10, 11, 12), pour insérer des insectes et/ou des nymphes et/ou des cocons, **caractérisée en ce qu'**une paroi basse comprend une rigole pour conduire un fluide vers une ouverture de sortie de fluide.

2. Cage (1) selon la revendication 1, dans laquelle la partie ouvrable comprend au moins un plateau (10, 11, 12), en particulier dans laquelle l'au moins un plateau (10, 11, 12) est amovible.

3. Cage (1) selon la revendication 2, dans laquelle l'au moins un plateau (10, 11, 12) est agencé pour fermer une ouverture dans un fond de la cage (1), au moins dans une position fermée du plateau (10, 11, 12).

4. Cage (1) selon l'une quelconque des revendications précédentes, dans laquelle la partie ouvrable (10, 11, 12) est accessible par un côté de la cage (1).

5. Cage (1) selon l'une quelconque des revendications précédentes, dans laquelle une paroi supérieure (4) comprend la partie au moins en partie translucide, qui est en particulier formée par une toile métallique ou un grillage.

6. Cage (1) selon l'une quelconque des revendications précédentes, dans laquelle au moins l'une des parois (3, 4, 5, 6, 7, 8) comprend une fente (13) pour insérer un dispositif de dépôt d'oeufs (14), comprenant au moins un orifice d'admission de gaz accessible par l'extérieur de la cage lorsque le dispositif (14) est placé dans la cage (1) ; et au moins un dispositif de refoulement de gaz, agencé à l'intérieur de la cage lorsque le dispositif est placé dans la cage.

7. Cage (1) selon la revendication 6, dans laquelle le dispositif de dépôt d'oeufs (14) comprend un matériau perméable aux gaz, tel qu'une éponge.

8. Cage (1) selon l'une quelconque des revendications précédentes, dans laquelle au moins l'une des parois (3, 4, 5, 6, 7, 8) comprend une entrée pour insérer un fluide.

9. Combinaison d'une pluralité de cages (1) selon l'une quelconque des revendications précédentes, et de sources d'éclairage, les cages et les sources d'éclairage étant agencées de sorte que la paroi au moins en partie translucide de chaque cage puisse être illuminée par une source d'éclairage.

10. Etagère pour contenir une pluralité de cages (1) selon l'une quelconque des revendications 1 à 8, comprenant au moins une source d'éclairage, dans laquelle la source d'éclairage est agencée de façon à pouvoir illuminer l'intérieur d'une cage à travers l'au moins une paroi au moins en partie translucide.

11. Etagère selon la revendication 10, comprenant un système de régulation pour réguler l'humidité et/ou la température.

12. Procédé d'élevage d'insectes, comprenant :
- la fourniture d'une cage (1) selon l'une quelconque des revendications 1 à 8 ;
- l'ajout d'une certaine quantité de nymphes dans la cage (1) ;
- le fait de faire éclore les nymphes à l'intérieur de la cage (1) ;
- la stimulation de mouches pour qu'elles s'accouplent afin de garantir la fertilité des oeufs ;
- la stimulation de mouches femelles pour qu'elles pondent leurs oeufs sur un dispositif de dépôt (14) de sorte qu'une récolte facile des oeufs soit possible.

13. Procédé selon la revendication 12, comprenant les étapes consistant à :
- après une certaine quantité de moments de récolte, laisser les mouches mourir dans la cage ;
- nettoyer la cage (1) en éliminant toutes les mouches mortes et les mouches vivantes restantes.

14. Procédé selon la revendication 13, comprenant la répétition de toutes les étapes avec une nouvelle quantité de mouches.
